# EUROPEAN PATENT APPLICATION

(11) **EP 4 814 084 A1**
(43) Date of publication of application: **30.09.2026**
(21) Application number: 24894174.2
(22) Date of filing: 20.11.2024
(51) Int. Cl.: C11B 9/00, G01N 33/497, A61B 5/16

(54) **EMOTION ENHANCER, METHOD FOR EVALUATING EMOTION, AND DEVICE FOR EVALUATING EMOTION**

(30) Priority: 20.11.2023 JP 2023196980
(71) Applicant: Shiseido Company, Ltd., Chuo-ku Tokyo 104-0061 (JP)
(72) Inventor: MATSUURA, Kazue, Tokyo 104-0061 (JP); KAWABATA DUNCAN, Keith James, Tokyo 104-0061 (JP); KATSUYAMA, Masako, Tokyo 104-0061 (JP); KAMEZONO, Rie, Tokyo 104-0061 (JP); MOTOYAMA, Akira, Tokyo 104-0061 (JP); KAGEYAMA, Ami, Tokyo 104-0061 (JP)
(74) Representative: Ter Meer und Partner mbB
(86) International application number: PCT/JP2024/041146
(87) International publication number: WO 2025/110183

(57) **Abstract**

It is an object of the invention to control emotion via odor components. It is another object of the invention to provide a method for evaluating emotion using an odor component as a marker. It was found that isovaleraldehyde is released as skin gas in a state with positive emotion, and further that isovaleraldehyde has the potential to propagate emotion to other humans, and that on this basis it becomes possible to provide an emotion enhancing agent that comprises isovaleraldehyde. Further provided is a method for evaluating emotion using isovaleraldehyde as a marker.

## Description

### FIELD

The present invention relates to an emotion enhancing agent that comprises isovaleraldehyde. It further relates to a method for evaluating emotion which comprises quantifying isovaleraldehyde, and to a device for carrying out the method.

### BACKGROUND

Odor is recognized by detecting odor molecules. Odor molecules bind to specific olfactory receptors that correspond to those odor molecules. When odor molecules bind to olfactory receptors, signal transduction takes place in the olfactory cells, causing depolarization. The olfactory nerve transmits a depolarization electrical signal to the brain, where it is recognized as an odor. Approximately 400 types of olfactory receptor genes are known in humans. Olfactory receptors form heterodimers, allowing detection of a variety of odor molecules. Electrical signals from olfactory cells are transmitted to the olfactory bulb of the brain, after which they are transmitted to the limbic system where the hippocampus and amygdala respond. Odors that have been recognized in the brain elicit emotions such as pleasure and discomfort, as well as triggering memories based on the odors. This is believed to be because sensing of odor molecules causes a response by the hippocampus, which controls memory.

Odor components such as citrus-based flavors and camphor are known to contribute to improved concentration and to help prevent drowsiness. By selecting such aroma components suited to individual preferences, it is possible to improve concentration and create a more relaxed state, and this concept is applied in the field of aromatherapy. At the same time, research is being conducted on odors that affect emotion regardless of personal preference. For example, it is known that characteristic body odors known as stress odors are released by humans who are undergoing psychological stress. Major components contributing to stress odor have been identified, and model compositions for body odor have been designed, such body odor model compositions containing sulfur compounds including one or more from among allylmercaptane and dimethyltrisulfide (PTL 1: Japanese Patent No. 7286624). Research has been conducted using such types of body odor model compositions as reference, to determine whether gas components produced by humans have any physiologically active effects similar to pheromones.

It has also been reported that the odor of human infants plays a part in feelings of love (NPL 1: PLoS ONE 11(5): e0154392 (2016)). With focus on the fact that odor molecules secreted by infants promote secretion of oxytocin, known as the "love hormone", this has led to compounds such as valeraldehyde, 1-octane, 2-undecanol, heptane, 2-heptene, 3-heptanol, butanal, 3-methylheptane and 2,3-butanediol being identified from among odor components secreted by infants, as oxytocin secretion promoters (PTL 2: Japanese Unexamined Patent Publication No. 2023-22459).

### [CITATION LIST]

### [PATENT LITERATURE]

[PTL 1] Japanese Patent Publication No. 7286624
[PTL 2] Japanese Unexamined Patent Publication No. 2023-22459

### [NON PATENT LITERATURE]

[NPL 1] PLoS ONE 11(5): e0154392 (2016)

### SUMMARY

### [TECHNICAL PROBLEM]

Human emotions vary greatly according to the individual, and are dependent on various factors including time, location and environment. Inducing positive emotions can help promote empathy and smoother communication. It is therefore an object of the present invention to identify odor components that can induce positive emotions, and to provide an emotion enhancing agent that allows control of an individual's own emotions or the emotions of persons around them. It is another object of the invention to provide a method for quantitative evaluation of emotions based on differences in components released from a human due to the emotions.

### [SOLUTION TO PROBLEM]

The present inventors have conducted diligent research on odor components released by humans, and on changes in emotion produced on the side where an odor component has been detected, and have completed this invention upon finding that odor components released in association with specific emotions contribute to transmission of those emotions. The present invention relates to the following:
[1] An emotion enhancing agent that comprises isovaleraldehyde.
[2] The emotion enhancing agent according to [1], wherein the emotion enhancing agent is a concentration improving agent or an anti-drowsiness agent.
[3] The emotion enhancing agent according to [1], wherein the emotion enhancing agent comprises 1.0 × 10⁻⁹ to 0.19% isovaleraldehyde.
[4] An aromatic composition comprising 1.0 × 10⁻⁹ to 0.19% isovaleraldehyde, and 0.5 to 99% of at least one component selected from the group consisting of triethyl citrate, propylene glycol, dipropylene glycol, dipropyleneglycol dibenzoate and ethanol, as a fragrance modifier.
[5] The aromatic composition according to [4], wherein the isovaleraldehyde is added as an emotion enhancing agent.
[6] The aromatic composition according to [4] or [5], wherein the aromatic composition is added to a food, cosmetic or fragrance cosmetic product.
[7] A method for evaluating change in positive emotion of a subject, comprising:
   a step of analyzing skin gas released from a portion of the body of the subject, and
   a step of evaluating emotion of the subject using the amount of at least one type of odor component in the skin gas as the marker.
[8] The method according to [7], wherein the skin gas is isovaleraldehyde.
[9] The method according to [8], wherein the emotion is evaluated by comparing the amount of isovaleraldehyde with a predetermined threshold value.
[10] The method according to [7], wherein the emotion of the subject is a positive emotion.
[11] The method according to [7], wherein the gas is obtained by placing part of a hand, foot or limb in a sealed space.
[12] The method according to [7], wherein the gas is obtained from the surrounding area where a hand has been placed.
[13] An emotion evaluating device that determines an emotional state based on a skin gas component, the emotion evaluating device comprising:
   an input unit to which data from analysis results from a skin gas analyzer is inputted;
   a storage unit that stores corresponding relationships between skin gas type and amount, and emotional state:
      a processing unit that determines the emotional state from the inputted data of the analysis results, and the corresponding relationships between skin gas type and amount and emotion, and
      an output unit that outputs the determined emotional state.
[14] The device according to [13], wherein the processing unit determines the emotional state using a learning unit that has been pre-trained using, as teacher data, skin gas types and their concentrations and information relating to their associated emotional states, and that outputs information relating to emotional state when information relating to skin gas type and concentration has been inputted.
[15] A system for evaluating an emotional state of a subject, the system comprising a device according to [13] and a terminal device which includes a display screen and a communication unit,
   wherein the output unit of the device is connected to a network via the communication unit, and
   the device and the terminal device communicate to display on the display screen of the terminal device the emotional state of the subject which has been determined by the device.

### [ADVANTAGEOUS EFFECTS OF INVENTION]

By using an emotion enhancing agent comprising isovaleraldehyde, it is possible to exhibit an effect of emotion enhancement, such as improvement of concentration or prevention of drowsiness. The emotional state can also be determined based on components of the skin gas.

### BRIEF DESCRIPTION OF DRAWINGS

Fig. 1 shows a Russell's circumplex model. The upper right quadrant may be considered to be a pleasant positive state with a high alertness level ("happy state"), and the lower right quadrant may be considered to be a pleasant positive state with a low alertness level ("relaxed state"). The upper left quadrant may be considered to be an unpleasant negative state with a high alertness level ("tense state"), and the lower left quadrant may be considered to be an unpleasant negative state with a low alertness level ("depressed state").
Fig. 2 shows the difference in peak area of a volatile component (isovaleraldehyde) in skin gas sampled with neutral emotion (left) and skin gas sampled with positive emotion (right). The peak area significantly increased with positive emotion.
Fig. 3 is a graph showing change in heart rate under odorless conditions and isovaleraldehyde conditions (approximately 5 × 10⁻⁹%).
Fig. 4 is a graph showing change in electromyographic activity of the zygomaticus major muscle under odorless conditions and isovaleraldehyde conditions (approximately 5 × 10⁻⁹%).

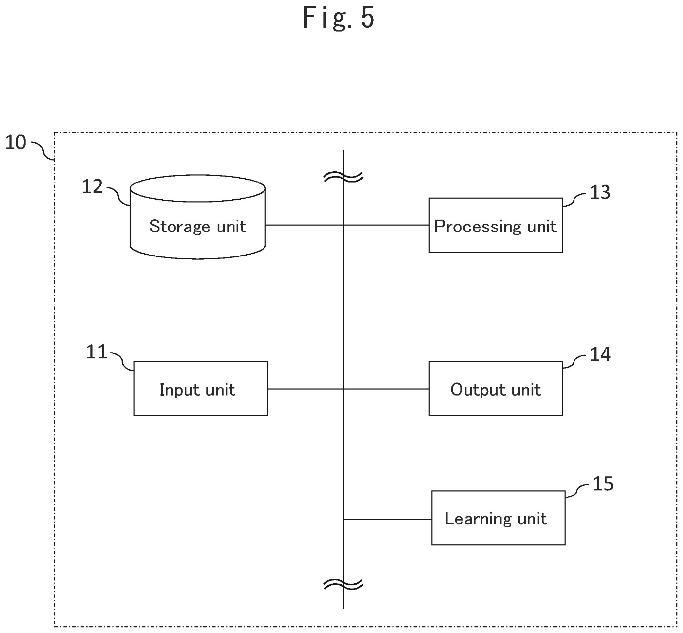

Fig. 5 shows a block diagram of an emotion evaluating device 10.

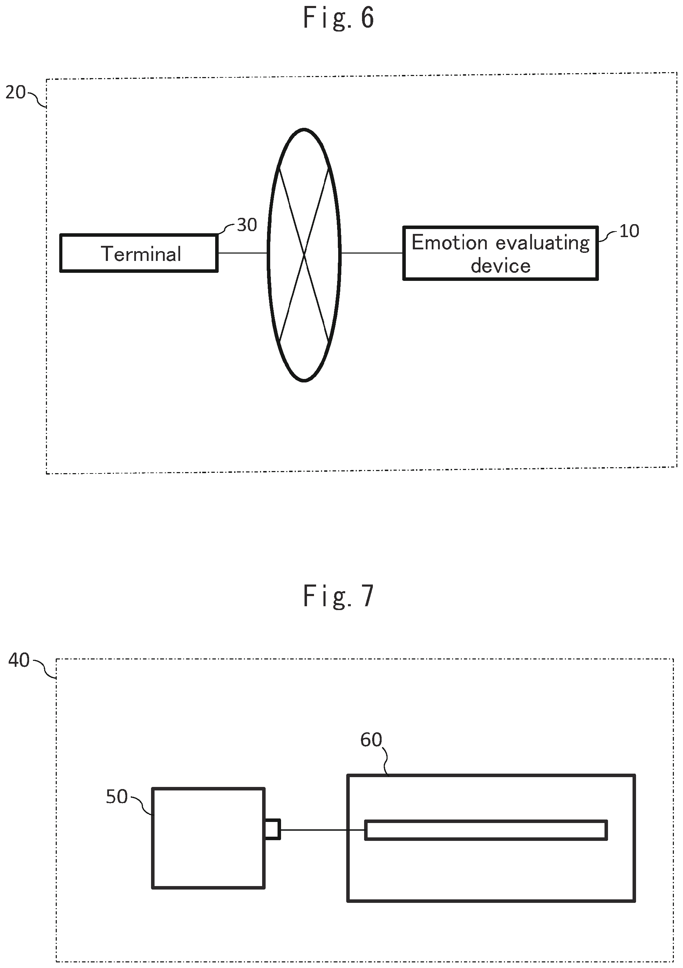

Fig. 6 shows a block diagram of an emotion evaluating system 20 comprising the emotion evaluating device 10.
Fig. 7 shows a block diagram of a skin gas analyzer 40.

### DESCRIPTION OF EMBODIMENTS

The present invention relates to an emotion enhancing agent that enhances emotion by odor components, to a method for evaluating emotion of a human subject by analyzing skin gas, and to a device that determines the emotion of a subject based on the types and concentrations of contents in skin gas.

### [Emotion enhancing agent]

An emotion enhancing agent is a formulation that causes a change in emotion either consciously or unconsciously, via the olfactory sense. Since such agents act via the olfactory sense, they may also be considered to be "olfactory emotion enhancing agents". Emotion enhancing agents include volatile organic compounds present in gas generated from skin. Isovaleraldehyde is an exemplary compound from the viewpoint of inducing positive emotion. Since the emotion enhancing agent of the invention induces positive emotion, it may also be referred to as a "vitality booster". Isovaleraldehyde has been identified, for the purpose of the invention, as a substance capable of transmitting positive emotion. Sending the odor of isovaleraldehyde can produce a natural smiling face and reduce stress. Sensing the odor of isovaleraldehyde can also improve concentration, while also increasing the level of alertness and thereby reducing drowsiness. The emotion enhancing agent of the invention can therefore be used as a concentration improving agent or as a drowsiness inhibitor. Emotions vary greatly depending on the individual and can thus often be difficult to express, but using the emotion enhancing agent of the invention allows positive emotions to be shared, thereby increasing empathy level and allowing smoother communication to be achieved.

Isovaleraldehyde is a compound represented by the following chemical formula: 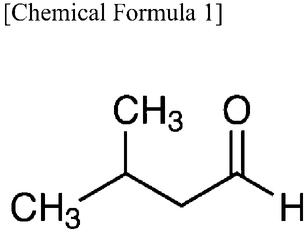 and it is also known as 3-methylbutanal. Among the structural isomers of valeraldehyde, isovaleraldehyde differs from normal-type valeraldehyde in its manner of branching. Valeraldehyde and isovaleraldehyde are both indicated as malodorous substances under the Offensive Odor Control Law. The odors exhibited by valeraldehyde and isovaleraldehyde differ depending on concentration, but valeraldehyde generally exhibits an unpleasant, irritating fruity aroma. Isovaleraldehyde, on the other hand, exhibits a characteristic clear odor at a concentration of about 0.05 ppbv to 0.1 ppbv by itself, and may also exhibit a cheese-like odor, sweat-like odor or malt-like odor. Valeraldehyde and isovaleraldehyde are also aroma components of fruit or sake, and both are used as food additives (aromatics).

The emotion enhancing agent of the invention may also be added to an aromatic composition. When added to an aromatic composition, the concentration may be appropriately selected according to the type of aromatic composition, with action on olfactory receptors being seen at a concentration of about 0.05 ppbv to 0.1 ppbv. The addition concentration in the aromatic composition will vary depending on the type of aromatic composition and is not particularly restricted so long as it is a concentration that is able to act on olfactory receptors, but an exemplary range is 1.0 × 10⁻⁹ to 0.19%. The concentration range may be one or more different ranges selected from among 1.0 × 10⁻⁹ to 1.0 × 10⁻⁸%, 1.0 × 10⁻⁸ to 1.0 × 10⁻⁷%, 1.0 × 10⁻⁷ to 1.0 × 10⁻⁶%, 1.0 × 10⁻⁶ to 1.0 × 10⁻⁵%, 1.0 × 10⁻⁵ to 1.0 × 10⁻⁴%, 1.0 × 10⁻⁴ to 1.0 × 10⁻³%, 1.0 × 10⁻³ to 1.0 × 10⁻²%, 1.0 × 10⁻² to 1.0 × 10⁻¹% and 1.0 × 10⁻¹ to 0.19%.

An aromatic composition according to the invention is a composition intended for fragrance, and which induces positive emotion, thus increasing the level of alertness and improving concentration, while also helping to prevent drowsiness, and it can therefore be used for smoothing communication. Aromatic compositions include fragrance products (such as perfumes, eau de parfum and body cologne), skin care cosmetics (base cosmetics such as cosmetic water, latexes, creams, essences and body milk, makeup cosmetics and antiperspirant deodorants), hair care cosmetics (shampoos, rinses, conditioners, treatments, tonics, hair restorers, hair coloring agents, hair styling agents and permanent agents), body cleansers (soaps, body soaps and cleaning sheets), bath additives, detergents (clothing detergents, softeners and spin finish agents), cleaning agents (for cleaning purposes, dishes, fibers, leather, residences, households, toilets or baths), oral cleaning agents (toothpaste, oral lavages and denture detergents), aromatic agents (space sprays, fiber sprays, aromatic agents for pets, residences, car inner spaces, toilets, baths, businesses and entertainment facilities, incense sticks, room fragrances, aroma candles and aroma diffusers), and foods (beverages, foods and supplements). The aromatic composition may be a water-soluble or oil-soluble liquid drug, a paste or a gel, or a powder, capsule or tablet. Various dosage forms can be produced by known methods. In particular, a volatile component may be encapsulated in polymer resin capsules, or it may be incorporated into the pores of a support such as silica, to produce long-lasting aroma.

From the viewpoint of use as an emotion enhancing agent, it is most preferred to use a fragrance product, or a personal care product such as a hair care product, body cleanser or cleaning sheet, or an aromatic agent that produces an aroma in a vehicle interior, indoor room or space. The aromatic agent may also be used in combination with a deodorant, as a deodorant aromatic agent. An aromatic agent for a vehicle interior may be used to prevent drowsiness while driving. The aromatic composition of the invention may be used as an aromatic agent in a shop, commercial facility, conference room, entertainment facility or entertainment device, to allow control of emotions. By using an aromatic composition for a pet product such as a collar, it is possible to improve relationships with pets. As one example, the aromatic composition of the invention may also exclude the aromatics or flavors for foods.

The aromatic composition may also contain other active components or various additives, in addition to the aroma component, depending on the purpose of use of the final product of the aromatic composition. Such active components will vary depending on the final product, but they may include bioactive substances, in the case of a cosmetic product or essence, or surfactants or enzymes in the case of a detergent. Examples of additives include water; organic solvents including alcohols such as ethanol, and triethyl citrate, glycerin, dipropylene glycol, propylene glycol and dipropyleneglycol dibenzoate; excipients; fats and oils; emulsifying agents; thickeners; antiseptic agents; and coloring agents. The organic solvent may be used to adjust the volatilization rate of isovaleraldehyde in the emotion enhancing agent, and it may also be referred to as a "fragrance modifier". The aromatic composition or emotion enhancing agent may include any organic solvent selected from among alcohols, triethyl citrate, glycerin, dipropylene glycol, propylene glycol and dipropyleneglycol dibenzoate, or a combination thereof, as a fragrance modifier, in the range of 0.5 to 99%. The concentration and type of fragrance modifier may be adjusted according to the volatility and/or long-lasting effect of isovaleraldehyde, and for example, it may be in a single range, or a plurality of contiguous ranges, selected from among 0.5 to 1%, 1% to 10%, 10 to 20%, 20 to 30%, 30 to 40%, 40 to 50%, 50 to 60%, 60 to 70%, 70 to 80%, 80 to 90%, 90 to 95%, 95% to 97% and 97% to 99%. These additives may be appropriately selected from among those commonly used in aromatic compositions. Examples of additives to be added to the aromatic composition include terpenes such as linalool.

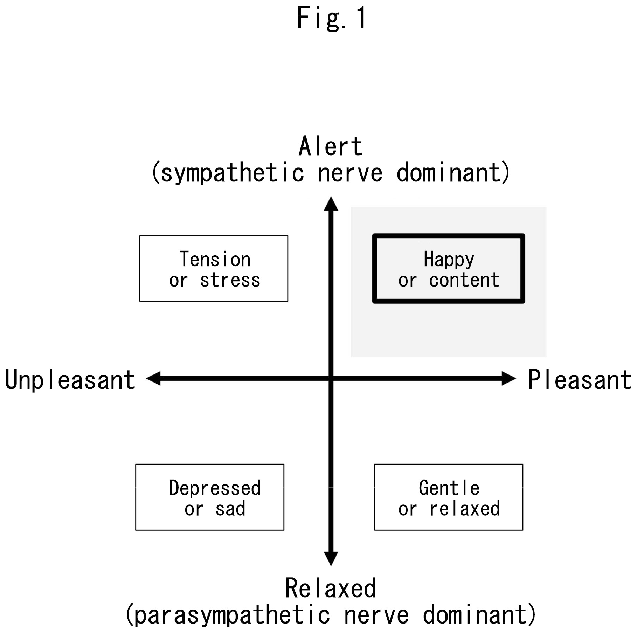

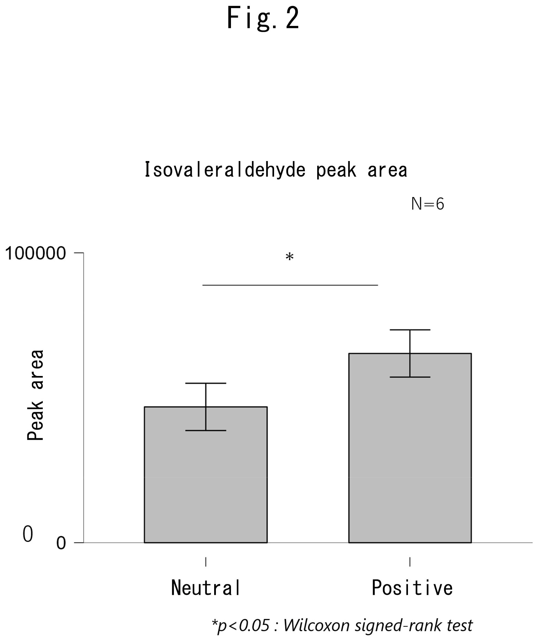

Isovaleraldehyde is an odor component in skin gas, being released particularly when experiencing positive emotion, and it can be detected *in vitro* (Fig. 2). A positive emotion is emotion acting on the region of the right side of the Russell's circumplex model, where emotions are classified based on unpleasant-pleasant and alertness-relaxation (Fig. 1). Therefore, "positive emotion" refers to favorable emotions such as interest, enjoyment, laughter, satisfaction, fulfillment, gentleness, calm, peace of mind and relaxation. Positive emotion can be induced by selecting and allowing viewing of entertainment videos that match individual preferences.

A neutral emotion, in contrast to a positive emotion, is an emotion near the intersection between the unpleasantness-pleasantness axis and the alertness-relaxation axis in the aforementioned Russell's circumplex model (Fig. 1), and it is emotion which is neither pleasant nor unpleasant, and neither alert nor relaxed. More preferably, the index of pleasantness and unpleasantness for neutral emotion is near 0 (Fig. 1). Positive emotions, and negative emotions in the bipolar region, are emotions in the region corresponding to the left half of the Russell's circumplex model. Emotion in a stress environment is emotion in the region of the Russell's circumplex model corresponding to alertness-unpleasantness.

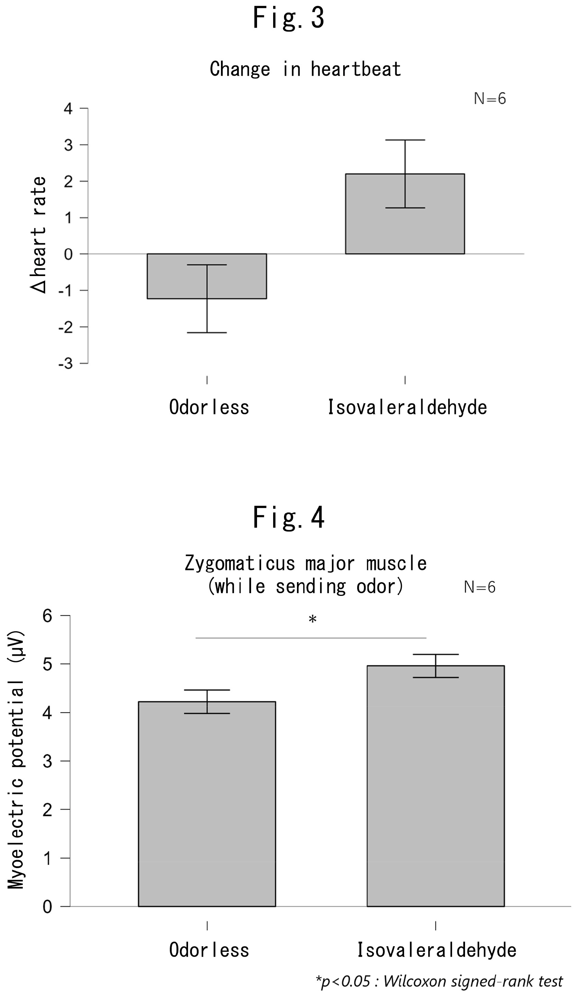

When skin gas emitted with positive emotion and skin gas emitted with neutral emotion are provided for organoleptic evaluation, skin gas with positive emotion exhibits a characteristic clear odor, while skin gas emitted with neutral emotion exhibits the body odor of the human. Skin gas and isovaleraldehyde with positive emotion both affect heart rate and respiration rate (Fig. 3).

Emotion affects the activity of facial expression muscles in an unconscious manner. Positive emotions are known to increase activity in the zygomaticus major muscle, while negative emotions are known to increase activity of corrugator muscles. In a subject that has sensed isovaleraldehyde odor, increased unconscious activity of the zygomaticus major muscle is observed (Fig. 4), and activity of the corrugator muscle can be reduced. Unconscious changes in zygomaticus major muscle activity can lead to increased activity of muscles surrounding the eye (orbital muscles), changes in wrinkles or facial features surrounding the eye (mouth angle, degree of opening or eye shape), or changes in degree of smiling, and such markers can also be evaluated using detection devices. In a subject where the odor of isovaleraldehyde has been sensed, it may have a subconscious effect on physiological markers. Such physiological markers include brain response, heart rate and heart rate variability, body heat, respiration rate, skin potential activity and hormone levels, as markers that can be evaluated using detection devices. Moreover, since positive emotion is elicited in a subject where isovaleraldehyde odor has been sensed, it is possible to increase resistance against stress reaction. These experimental results demonstrate that isovaleraldehyde can be used as an emotion enhancing agent.

The emotion enhancing agent or aromatic composition of the invention can be applied at a timing in which a specific emotion (negative or positive emotion, for example) is detected in speech or non-verbal communication, during conversation between two or more individuals. The emotion enhancing agent or aromatic composition containing isovaleraldehyde is supplied by spraying at such a timing. By supplying the emotion enhancing agent or aromatic composition into a space by spraying, the emotion enhancing agent or aromatic composition containing isovaleraldehyde acts on individual emotions and smooths communication. There are no particular restrictions on the timing for spraying of the emotion enhancing agent, and for example, it may be periodic spraying whenever a specific emotion has been detected, regardless of the emotion. Since it is sufficient for the aroma to be released to a person in the space, it may be distributed by being supported on a carrier capable of supporting the emotion enhancing agent or aromatic composition in a releasable manner, in addition to spraying. Changes in facial expression and emotion can be detected and evaluated based on activity of facial expression muscles, and on techniques for estimating facial expression and emotion based on machine learning.

Reactivity with isovaleraldehyde can also be used as a marker to allow evaluation of the ease of transition toward positive emotion. By reacting with isovaleraldehyde, it is possible to produce transition from negative emotions, i.e. emotions corresponding to the region at the left half of the Russell's circumplex model, and more specifically emotions in a stressful environment (emotions in the region corresponding to alertness-unpleasantness in the Russell's circumplex model) or depressed emotions (emotions in the region corresponding to relaxation-unpleasantness in the Russell's circumplex model), toward positive emotions. By using reactivity with isovaleraldehyde as the marker, therefore, it is possible to determine a condition of stress resistance or resistance to depression. Reactivity for isovaleraldehyde can be determined by measurement of physiological markers or by questionnaire, after the odor of isovaleraldehyde has been sensed, during which time an inert gas such as nitrogen gas may be used as a control.

### [Method for evaluating emotion]

Another aspect of the invention relates to a method for evaluating emotion of a human subject by analysis of skin gas. This method includes a step of analyzing skin gas, a step of quantifying the odor components in the skin gas, and a step of evaluating the emotion of a subject using the amount of specific odor components, the amount of change (especially increase), the ratio, or the rate of change (especially increase rate) as a marker. Isovaleraldehyde may be selected as the odor component. If the isovaleraldehyde amount, increase, ratio or increase rate is high, then it will be possible to determine that the subject has a positive emotion. By setting the isovaleraldehyde threshold it is possible to identify an emotion in comparison to the threshold. Such a threshold value can be appropriately set according to the method of sampling and analysis of skin gas.

Skin gas sampling and analysis may be carried out by any desired method. As an example, released components which may be gas or volatile substances can be detected by sampling gas surrounding the skin. When gas is to be sampled from a hand, for example, the entirety of the hand may be covered with an air-impermeable bag (for example, a plastic bag) with sealing at the wrist section. The bag preferably has an openable and closeable sampling hole that allows gas in the bag to be removed. The gas in the bag is preferably air, or it may be replaced with an inert gas (such as nitrogen gas). After a specified time period has elapsed, the gas in the bag may be transferred through the sampling hole to a separate container (such as a storage container). The gas in the storage container is supplied to a chromatography apparatus for analysis of the components in the gas.

### [Emotion evaluating device]

Another aspect of the invention may relate to an emotion evaluating device 10 that carries out a method for evaluating emotion. The emotion evaluating device 10 exhibits its function by a computer comprising a storage unit 12, an input unit 11, a processing unit 13 and an output unit 14. It may also include a learning unit 15 either instead of the storage unit 12 and processing unit 13, or in addition to the storage unit 12 and processing unit 13.

More specifically, the emotion evaluating device 10 comprises the following:
an input unit 11 to which data from analysis results from a skin gas analyzer is inputted;
a storage unit 12 that stores corresponding relationships between skin gas type and amount, and emotion:
   a processing unit 13 that determines an emotion from the inputted data of the analysis results, and the corresponding relationships between skin gas type and amount and emotion, and
   an output unit 14 that outputs the determined emotion. According to yet another example, the emotion evaluating device 10 comprises the following:
      an input unit in which data from analysis results from a skin gas analyzer is inputted;
      a learning unit that is pre-trained on skin gas types and concentrations and information relating to associated emotions, as teacher data, and outputs information relating to an emotion, when information relating to a skin gas type and concentration has been inputted; and
      an output unit that outputs the determined emotional state. Data from the analysis results are inputted from the skin gas analyzer 40 through the input unit 11. The inputted data may be directly processed by the processing unit of the emotion evaluating device 10, or it may be processed after having been stored in the storage unit 12 and read by the processing unit 13. The storage unit 12 may also pre-store corresponding relationships between skin gas type and amount, and emotion. The corresponding relationships between skin gas type and amount and emotional state may also be provided by a trained learning unit 15. The processing unit 13 reads out corresponding relationships between emotions and the types and concentrations of contents in skin gas which are stored in the storage unit, and compares these with the inputted data to determine the emotional state of the subject. As another example, the processing unit reads out from the trained learning unit 15 and inputs into the learning unit 15 information relating to skin gas types and concentrations, to allow output of the emotion-related information. Emotions of a subject may include positive emotions, negative emotions, tension and relaxation. Lying is associated with a tense state, and therefore identification of skin gas representing a tense state allows lying to be determined. By measuring the isovaleraldehyde concentration in real time it is possible to determine a state of curiosity or interest. The skin gas may be determined as a single type, or an emotional state may be determined by the relationship between several different types of skin gases and their amounts. The results of sampling and analyzing skin gas in real time may be inputted through the input unit 11 of the emotion evaluating device 10, or the skin gas may be separated out over a predetermined time period and provided for analysis by batch processing, and the results inputted through the input unit 11 of the emotion evaluating device 10.

According to one embodiment, an emotion may be determined by the processing unit 13 by comparison with the threshold for the amount of at least one component stored in the storage unit 12. The determined emotion is stored in the storage unit 12 and outputted through the output unit 14. More preferably, the amount of isovaleraldehyde is compared with the threshold, with an emotion being determined to be a positive emotion when the amount is at least a predetermined concentration.

According to another embodiment, the processing unit 13 of the emotion evaluating device 10 may determine an emotion using a learning unit 15 that has been pre-trained using, as teacher data, skin gas types and concentrations and information relating to associated emotions. In such cases, the emotion evaluating device 10 may comprise a learning unit 15 that has been pre-trained using, as teacher data, skin gas types and concentrations, and associated information relating to emotions. The learning unit has been pre-trained so as to output an specific emotional state when the type of skin gas and its concentration have been inputted.

The storage unit 12 has a portable storage device which may be a memory device such as a RAM, ROM or flash memory, a fixed disk device such as a hard disk drive, or a flexible disk or optical disk. The storage unit 12 stores data and instructions inputted through the input unit 11, one or more component amounts in the inputted data, and the threshold for determining the relationship between the amount of that component and an emotion. The threshold value data may include multiple threshold values, or the data may be stored as a correspondence table. The storage unit 12 may also store, in addition to the computation processing results obtained by the processing unit 13, a program or database to be used for various computer processing operations, and it may also store the program for the learning unit. The computer program may be on a computer readable recording medium such as a CD-ROM or DVD-ROM, or it may be installed via the internet. The computer program is installed in the storage unit 12 using a commonly known setup program, for example.

The input unit 11 includes an interface. The interface may be connected to an operating unit such as a keyboard or mouse, or a communication unit such as a LAN or port, or an external memory unit such as a CD-ROM, DVD-ROM, BD-ROM or memory stick. The amount of specific components may also be inputted through an operating unit. Instructions for processing by the processing unit 13 can be provided from the input unit 11 via an operating unit.

The processing unit 13 executes computation processing according to the program stored in the storage unit 12. Computation processing is carried out by a central processing unit (CPU) in the processing unit 13. The CPU comprises a functional module that controls the input unit 11, storage unit 12, learning unit 15 and output unit 14, and is able to control various control operations. Each of the units may be constructed by independent integrated circuits, microprocessors and firmware. The information generated after each processing by the processing unit 13 may be stored in the storage unit 12 first, or it may be used directly for the subsequent processing.

The output unit 14 is constructed so as to output the emotion generated upon computation processing by the processing unit 13. The output unit 14 may be output means such as a display device with a liquid crystal display that directly displays the computation processing results, or a printer, or it may be an interface unit for output to an external memory unit or output to a network.

The learning unit 15 learns relationships between input data relating to types and concentrations of skin gas, and information relating to associated emotions, using a known machine learning technique such as deep learning. It is possible to analyze skin gas sampled in a variety of environments, including positive emotional states, relaxed environments, negative emotional states and stressful environments, and to train on skin gas types and concentrations and information relating to associated emotions. Deep learning is a type of machine learning using a multilayer structure neural network composed of an input layer, interlayer and output layer. A feature vector for detection information is inputted into each node of the input layer. Each of the nodes of the interlayer outputs the sum of the values obtained by multiplying the feature vector outputted from each node of the input layer by its corresponding weight, and the output layer outputs the sum of the values obtained by multiplying the feature vector outputted from each node of the interlayer by its corresponding weight. While adjusting each weight, the learning unit 15 is trained so as to reduce the difference between the output value from the output layer and the data for emotion. The input data inputted into the learning unit 15 includes the types and concentrations of one or more different skin gases. Chromatography, providing information including the type of skin gas and its concentration, may be directly inputted as input data, or a skin gas component may be identified and inputted together with its concentration. The trained learning unit 15 is held in the storage unit 12 and can be carried out by the functional module of the processing unit 13.

The emotion evaluating device 10 of the invention may also construct the emotion evaluating system 20 on a network and in combination with a terminal 30. The emotion evaluating device 10 may exist on a server, with the input unit 11 and output unit 14 connected to the network via the interface unit. The learning unit used by the emotion evaluating device 10 may also be disposed on the outside via a server, to allow evaluation of emotion via communication.

Another aspect of the invention relates to a program or control method in which the processing described above is carried out by the emotion evaluating device 10. The program or control method comprises the following commands or steps which are caused to be carried out by the processing unit:
storing in the storage unit 12 the data for the analysis results inputted through the input unit 11,
reading out the corresponding relationship between an emotion and the amount of one or more components stored in the storage unit 12,
determining the emotion from the data for the analysis results and the corresponding relationship between the emotion and the amount of the one or more components,
storing the determined emotion in the storage unit 12, and
outputting the determined emotion to the output unit 14. The processing unit 13 may determine emotion using a learning unit that has been pre-trained to output an emotional state when a particular type of skin gas and its concentration are inputted.

The analysis results for the skin gas analyzer 40 are inputted into the input unit 11 of the emotion evaluating device 10 of the invention. The skin gas analyzer 40 may also be included as part of the emotion evaluating device 10. The skin gas analyzer 40 comprises a skin gas sampling unit 50 and a skin gas analyzer 60. The skin gas collected by the skin gas sampling unit 50 is supplied to the skin gas analyzer 60 to allow the contents and concentration of the skin gas to be determined.

The location where the skin gas is sampled may be, for example, a hand (especially a hand palm), flank, foot (especially a foot sole), back, head, oral or cavity, or exhalation gas. For example, when skin gas is to be sampled from a hand, the entirety of the hand may be covered with an air-impermeable bag (for example, a plastic bag) with sealing at the wrist section. The bag preferably has an openable and closeable sampling hole that allows gas in the bag to be removed. The gas in the bag is preferably air, or it may be replaced with an inert gas (such as nitrogen gas).

The skin gas analyzer 60 may be any analyzer that can analyze skin gas. A chromatography detector may be used as the analyzer, but in particular a gas chromatography-mass spectrometer (GC-MS) may be used from the viewpoint of sensitivity.

According to one embodiment, the device for evaluating emotion of a subject according to the invention is device for evaluating emotion of a subject that comprises the following:
a skin gas sampling unit 50 that samples skin gas from a portion of the body of the subject;
a skin gas analyzer 60 that analyzes the sampled skin gas; and
an emotion evaluating device 10 to which is inputted the analysis results from the skin gas analyzer 60 through the input unit 11;
wherein the emotion of the subject can be evaluated in real time or by batch processing.

All of the publications mentioned throughout the present specification are incorporated herein in their entirety by reference.

The Examples of the invention described below are intended to serve merely as illustration and do not limit the technical scope of the invention. The technical scope of the invention is limited solely by the description in the Claims. Modifications of the invention, such as additions, deletions or substitutions to the constituent features of the invention, are possible so long as the gist of the invention is maintained.

### EXAMPLES

### Example 1: Analysis of skin gas

### Sampling of skin gas

Skin gas sampling bags comprising connecting parts were attached to the hands of 6 test subjects, and sealing was confirmed. Air was suctioned from each connecting part, and then approximately 0.5 L of nitrogen gas was filled in. After attaching a skin gas sampling bag made of a material with a high gas barrier property, skin gas was sampled for 20 minutes while in a neutral emotional state evoked by reading a business journal. After then attaching a sampling bag in the same manner, moving images selected to match the preference of the test subject were shown for 20 minutes, in order to obtain skin gas with positive emotion.

### Analysis of skin gas

The obtained skin gas sample was transferred from the connecting part to a skin gas sampling bag prepared from a material with a high gas barrier property, and stored there. The skin gas in the storage bag was analyzed by gas chromatography mass spectrometry. As a result of analyzing the skin gas with positive emotion, isovaleraldehyde was identified as a component exhibiting a characteristic clear odor, and the peak area was compared. The results are shown in Fig. 2.

### Example 2: Evaluation of positive skin gas component or isovaleraldehyde

Isovaleraldehyde gas and odorless nitrogen gas as a control were supplied for organoleptic evaluation. The responses of heartbeat and facial expression muscles were measured for 6 organoleptic evaluators when sensing different odors.

### Effect on heartbeat

Electrodes of a Polymate 1000 (AP1532, product of Miyuki Giken Co., Ltd.) were attached at 3 points: below the right clavicle, below the left clavicle and on the lower chest, and an electrocardiogram signal was recorded at the points where isovaleraldehyde was approximately 5 × 10⁻⁹% and odorless nitrogen gas (control) was inhaled, examining the change in heart rate at those times. The results are shown in Fig. 3.

Since a greater change in heart rate was observed under isovaleraldehyde conditions than under odorless conditions, this suggested the possibility that alertness level was subconsciously increased by the aroma.

### Effect on facial expression muscles

Electrodes of a Polymate 1000 (AP1532, product of Miyuki Giken Co., Ltd.) were attached to the zygomaticus major muscle, and the muscle activity was examined after inhaling nitrogen gas and approximately 5 × 10⁻⁹% isovaleraldehyde. The measurement results are shown in Fig. 4. Since the activity level of the zygomaticus major muscle significantly increased under isovaleraldehyde conditions compared to the odorless conditions, this indicates the possibility that pleasant emotion was subconsciously induced by the aroma.

## Claims

1. An emotion enhancing agent that comprises isovaleraldehyde.

2. The emotion enhancing agent according to claim 1, wherein the emotion enhancing agent is a concentration improving agent or an anti-drowsiness agent.

3. The emotion enhancing agent according to claim 1, wherein the emotion enhancing agent comprises 1.0 × 10⁻⁹ to 0.19% isovaleraldehyde.

4. An aromatic composition comprising 1.0 × 10⁻⁹ to 0.19% isovaleraldehyde, and 0.5 to 99% of at least one component selected from the group consisting of triethyl citrate, propylene glycol, dipropylene glycol, dipropyleneglycol dibenzoate and ethanol, as a fragrance modifier.

5. The aromatic composition according to claim 4, wherein the isovaleraldehyde is added as an emotion enhancing agent.

6. The aromatic composition according to claim 4 or 5, wherein the aromatic composition is added to a food, cosmetic or fragrance cosmetic product.

7. A method for evaluating changes in positive emotion of a subject, comprising:
a step of analyzing skin gas released from a portion of the body of the subject, and
a step of evaluating emotion of the subject using the amount of at least one type of odor component in the skin gas as the marker.

8. The method according to claim 7, wherein the skin gas is isovaleraldehyde.

9. The method according to claim 8, wherein the emotion is evaluated by comparing the amount of isovaleraldehyde with a predetermined threshold value.

10. The method according to claim 7, wherein the emotion of the subject is a positive emotion.

11. The method according to claim 7, wherein the gas is obtained by placing part of a hand, foot or limb in a sealed space.

12. The method according to claim 7, wherein the gas is obtained from the surrounding area where a hand or foot has been placed.

13. An emotion evaluating device that determines an emotional state based on a skin gas component, the emotion evaluating device comprising:
an input unit to which data from analysis results from a skin gas analyzer is inputted;
a storage unit that stores corresponding relationships between skin gas type and amount, and emotional state:
a processing unit that determines the emotional state from the inputted data of the analysis results, and the corresponding relationships between skin gas type and amount and emotion, and
an output unit that outputs the determined emotional state.

14. An emotion evaluating device that determines an emotional state based on components in skin gas, the emotion evaluating device comprising the following:
an input unit in which data from analysis results from a skin gas analyzer is inputted;
a learning unit that is pre-trained on skin gas types and concentrations and information relating to associated emotions, as teacher data, and outputs information relating to an emotion, when information relating to a skin gas type and concentration has been inputted; and
an output unit that outputs the determined emotional state.

15. A system for evaluating an emotional state of a subject, the system comprising a device according to claim 13 or 14 and a terminal device which comprises a display screen and a communication unit, wherein:
the output unit of the device is connected to a network via the communication unit, and
the device and the terminal device communicate to display on the display screen of the terminal device the emotional state of the subject which has been determined by the device.
